# EUROPEAN PATENT SPECIFICATION

(11) **EP 2 702 578 B1**
(45) Date of publication and mention of the grant of the patent: **10.08.2016**
(21) Application number: 12777381.0
(22) Date of filing: 25.04.2012
(51) Int. Cl.: G08C 19/12

(54) **WIRELESS SENSOR READER**
DRAHTLOSER SENSOR-LESER
LECTEUR À CAPTEUR SANS FIL

(30) Priority: 25.04.2011 US 201161478647 P; 19.03.2012 US 201213423693
(43) Date of publication of application: 05.03.2014
(60) Divisional application: 16000758.9
(73) Proprietor: Endotronix, Inc., East Peoria, IL 61611-2018 (US)
(72) Inventor: NAGY, Michael, Lawrenceville GA 30043 (US); ROWLAND, Harry, Plainfield IL 60585 (US); WATKINS, Roger, Dunlap IL 61525 (US); SUNDARAM, Balamurugan, Dunlap, IL 61525-9558 (US)
(74) Representative: Müller-Boré & Partner Patentanwälte PartG mbB
(86) International application number: PCT/US2012/034979
(87) International publication number: WO 2012/149008

(56) References cited:
- US-A1- 2009 189 741
- US-A1- 2009 189 741
- US-A1- 2010 308 974
- US-A1- 2010 308 974
- US-B2- 7 566 308

## Description

### FIELD OF INVENTION

The present invention generally relates to an apparatus and device for measuring a wireless signal from a sensor.

### BACKGROUND

US 2009/189741 A1 discloses a wireless sensor reader adapted to transmit an excitation pulse to cause a wireless sensor to generate a ring signal. The wireless sensor reader receives and amplifies the ring signal and sends the signal to a phase-locked loop. A voltage-controlled oscillator in the phase-locked loop locks onto the ring signal frequency and generates a count signal at a frequency related to the ring signal frequency. The voltage-controlled oscillator is placed into a hold mode where the control voltage is maintained constant to allow the count signal frequency to be determined. US 2010/308974 A1 discloses a wireless sensor reader transmitting a narrow-band, fixed frequency excitation pulse to cause the wireless sensor to generate a ring signal.

Wireless sensor and reader systems may be designed to wirelessly monitor the status of a remote sensor. Some such wireless systems include a sensor that transduces a physical parameter into a signal frequency. A reader is then configured to receive and measure the frequency of the sensor signal.

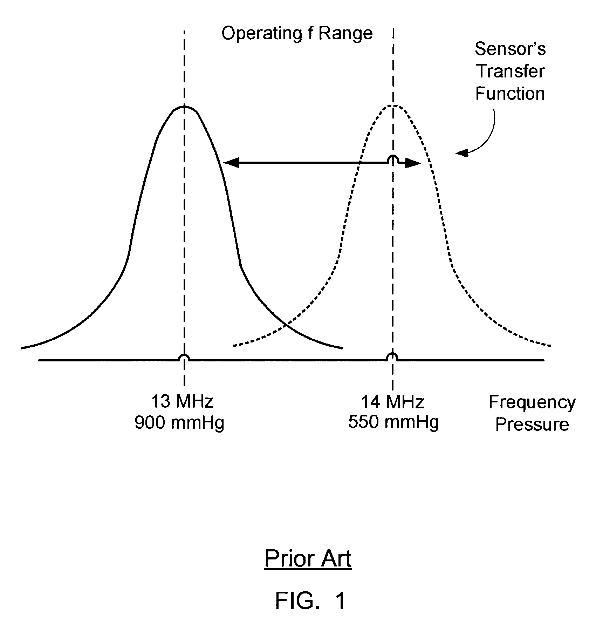

FIG. 1 illustrates an example of an operational frequency bandwidth of a wireless sensor/reader system and the corresponding parameter. As shown, the corresponding parameter is pressure, however it will be appreciated that the concept described herein may apply to any transduced parameter. The exemplary frequency range of the illustrated wireless sensor is from 13 to 14 MHz, which corresponds to absolute pressures of 550-900 mmHg. In the example shown in FIG. 1, frequency is inversely proportional to pressure.

In wireless sensor/reader systems, the sensor may be stimulated by a transmit pulse from a reader, causing the sensor to emit a ring back or "ring" signal at its resonant frequency once that stimulus is removed. The reader may measure the frequency of the ring signal and use a calibration table or formula to determine the sensed pressure.

The ring signal, as received at the reader, may be low power and may decay very quickly, particularly if the distance between sensor and reader is great. This is a problem with all similar wireless sensor systems, whether the systems utilizes a transmit signal that is fixed or swept. Other types of wireless sensor systems, such as those based on grid-dip techniques, may require a relatively long time and many transmit cycles to identify the sensor's resonance frequency, especially when the possible range of resonance frequencies is large.

Some wireless reader/sensor system designs require a gauge pressure reading, meaning pressure relative to local atmospheric pressure. In such designs, however, the sensor is often located at a position where it cannot access atmospheric pressure and thus cannot directly deliver a gauge pressure reading. For example, a blood pressure sensor implanted in the pulmonary artery is not capable of directly accessing atmospheric pressure. To deal with certain medical conditions, clinicians typically wish to know the gauge pressure of the pulmonary artery across a range of 100 mmHg. However, the implanted sensor has no way of knowing what the local atmospheric pressure is. In other words, the implanted sensor is only capable of sensing absolute pressure.

One solution is to place an ambient pressure sensor in the reader. The reader then measures absolute pressure from the implanted sensor, as well as absolute atmospheric ambient pressure from its ambient pressure sensor, and subtracts the ambient pressure from the absolute pressure to obtain gauge pressure.

The example in FIG. 1 illustrates a pressure range between 550-900 mmHg absolute. Ambient pressures in the inhabited regions of earth typically range from 550-800 mmHg absolute. Thus, to measure 0-100 mmHg gage, a sensor's absolute range must go from 550 mmHg (lowest ambient 550 mmHg plus lowest gauge 0 mmHg) to 900 mmHg (highest ambient 850 mmHg plus highest gauge 100 mmHg).

Therefore, there is a need to measure the frequency of a weak signal where the signal's full scale range is wide, but where only a small subset of that full range is used for any individual measurement.

Regardless of the method used to determine the sensor signal frequency, various circuits within the reader must be adapted or tuned to capture the maximum amount of energy in the sensor signal without capturing unwanted energy from sources other than the sensor, such as natural or man-made noise. For example, the reader's receiver antenna and internal filters, such as analog or digital filters, may be tuned to a passband that passes any possible frequency at which the sensor might resonate and rejects all frequencies outside that passband. However, widening the passbands of antennas and filters can cause problems, including higher attenuation, lower signal-to-noise ratios, and increased susceptibility to unwanted interfering signals.

Fixed frequency systems have difficulty overcoming these problems. Some swept frequency systems may attempt to overcome the problems by constantly retuning the receivers and filters to match the instantaneous frequency being transmitted. This, however, usually requires significant additional circuitry and processing.

Therefore, an improved method and apparatus are needed.

### SUMMARY

The invention relates to a wireless sensor reader having the features of claim 1. Preferred embodiments are defined in the dependent claims.

A reader device is provided to interface with a wireless sensor. The reader emits a short pulse of energy or a short burst of radio frequency energy to cause the wireless sensor to ring. Immediately after the transmission, the reader receives and amplifies the sensor signal, then sends the signal to a phase-locked loop ("PLL") that locks to the sensor ring frequency. Once the PLL has locked to the ring frequency, the PLL's voltage controlled oscillator ("VCO") is placed in a hold mode to maintain the VCO frequency at the locked frequency. The VCO frequency is counted to determine the sensor resonant frequency.

The reader may include a device, such as a second sensor, to determine a set of possible frequency values of the ring signal. The components of the reader device may be tuned to the set of possible frequency values that are identified.

### BRIEF DESCRIPTION OF THE DRAWINGS

Objects and advantages together with the operation of the invention may be better understood by reference to the detailed description taken in connection with the following illustrations, wherein:
FIG. 1 is a graph of an operational frequency bandwidth of a sensor and corresponding parameter;

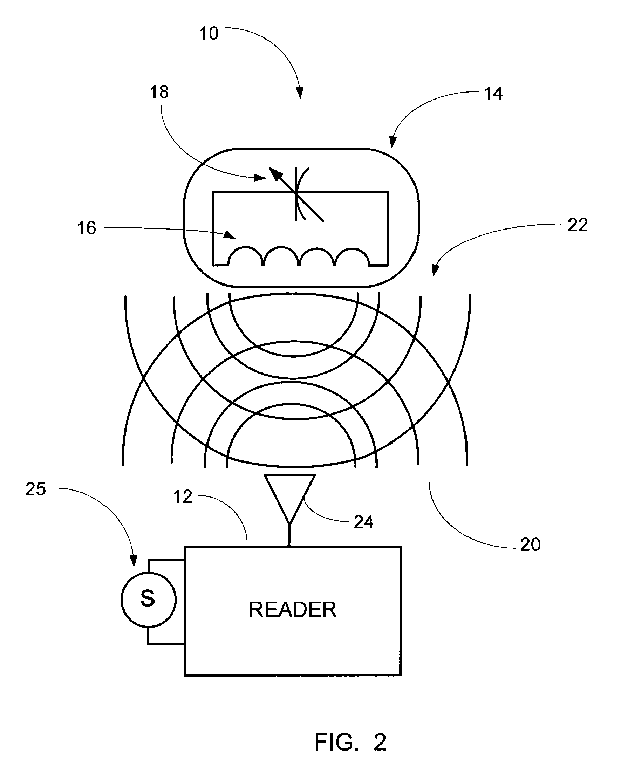

FIG. 2 is an embodiment of a wireless sensor system; and
FIG. 3 is a graph of an operational frequency bandwidth of a sensor and corresponding parameter and bandpass window.

### DETAILED DESCRIPTION

Reference will now be made in detail to exemplary embodiments of the present invention, examples of which are illustrated in the accompanying drawings. It is to be understood that other embodiments may be utilized and structural and functional changes may be made without departing from the respective scope of the present invention.

A wireless system 10 is generally provided. The wireless system 10 may include a wireless reader 12 and a wireless sensor 14. The wireless sensor 14 may be a passive device, such as a device comprising a capacitor 16 and an inductor 18, or an active device. The wireless sensor 14 may be implantable, such as implantable into a living being. For example, the wireless sensor 14 may be implanted in a human body to monitor a condition or parameter within the human body.

The reader 12 may be configured to transmit an excitation pulse 20 to excite the sensor 14. The excitation pulse 20 may cause the sensor 14 to ring or emit a ring signal 22 at its resonant frequency. The resonant frequency of the sensor 14 may vary based on a parameter sensed by the sensor 14. The reader 12 may measure the frequency of the ring signal 22 and determine the sensed parameter. For example, the reader 12 may utilize a formula, lookup table or calibration table to determine the sensed parameter.

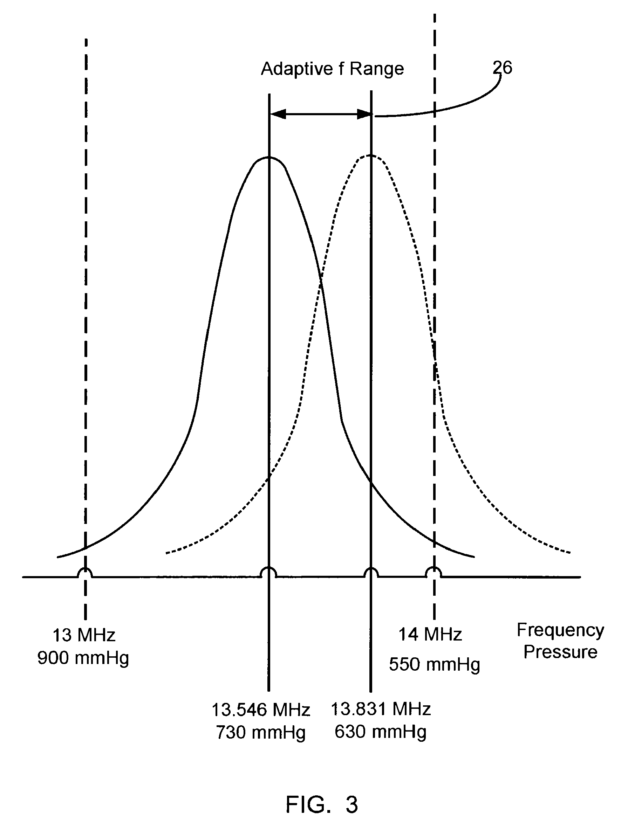

The reader 12 may include a receiver to receive the ring signal 22 from the sensor 14. The receiver may comprise an antenna 24 or any other signal receiving device. The receiver may further include one or more filters, such as for example analog or digital filters, to filter the signal 22 received from the sensor 14. The filters may be tuned to a passband to allow a desired frequency bandwidth to be received by the reader 12. The passband may be narrowed to pass only a frequency band that corresponds to a specific parametric range of interest 26, shown in figure 3.

Exemplary embodiments described herein may make reference to monitoring and sensing a specific parameter, such as pressure. It will be appreciated, however, that the systems and methods set forth herein may be applied to any measured or sensed parameter, such as pressure, temperature, or any other parameter.

By way of a non-limiting example, a wireless system 10 adapted to sense a pressure, such as blood pressure, may include filters to narrow the passband window 26 to only receive frequencies that correspond to pressures within a 100 mmHg gauge pressure range. An example of this passband range 26 is illustrated in FIG. 3. The frequencies that correspond to pressures within a 100 mmHg gauge pressure range may be a "passband window" or "window of interest" 26 of the frequencies that provide the optimal or most valuable data. It will be appreciated, however, that the passband window 26 may correspond to any appropriate range of the sensed parameter.

The spectral location of the passband window 26 within the total range of absolute pressure may vary to capture the desired data. For example, the location of the window 26 may be determined based on the ambient pressure at the time the reader 12 is receiving the ring signal 22 from the sensor 14. To that end, the reader 12 may include an ambient sensor 25, such as an ambient pressure sensor, to sense an ambient condition, such as pressure. The ambient sensor 25 may be embedded in or located on the reader 12. The ambient sensor 25 may also be located away from the reader 12, such as part of another device or system that communicates its ambient reading to the reader 12 or to a third party processor, for determining the location of the passband window 26.

As shown in the graph illustrated in FIG. 3, the passband window 26 may be optimally located based on the ambient pressure measured by the reader's ambient pressure sensor 25. For example, in an embodiment where the sensor is a wireless pressure sensor implanted in the pulmonary artery of a human being, the pressure range of interest is 0-100 mmHg above ambient. Therefore, the Reader's processor would be programmed to locate a passband window 26 such that its edges are at frequencies corresponding to the ambient pressure reading, and a pressure that is 100 mmHg greater than the ambient pressure reading, as shown in Figure 3. Accordingly, the reader 12 may tune its antenna 24, as well as its internal circuits and algorithms, to focus the passband window 26 near the ambient pressure.

In an embodiment, a wireless sensor 14 may be implanted into a human being located at relatively high altitude, for example an altitude having an ambient pressure near 630 mmHg absolute. The pressure range of interest may therefore be 630-730 mmHg absolute, corresponding to a frequency passband window 26 of 13.831 - 13.546 MHz. The reader 12 may measure the ambient pressure using its ambient pressure sensor 25. The reader 12 may then determine, from the ambient pressure measurement, the subset of the full-scale frequency range that will contain the remote sensor's frequency. The reader 12 may then tune its receiver, such as the antennas 24, filters, amplifiers, other circuits, or algorithms, to pass the desired subset and block the unwanted portion of the range. For example, the reader 12 may increase the Q of its receiving antenna by narrowing its bandwidth to match the frequency window 26. Additionally, the reader 12 may increase the gain and signal-to-noise ratio of one or more amplifiers in the receive chain by tuning them to the passband window 26. The reader 12 may also tune filters in the receive chain to match the passband window 26, and thus filter out any noise or interference outside the passband window 26. The reader 12 may take numerous pressure readings from the sensor and average them (in its own embedded processor or in a remote processor) to further improve accuracy. The averaging processor may implement an algorithm by which all readings that fall outside the passband window 26 are considered spurious outliers and are not included in the average.

This system and method, as described, provide several advantages over known systems and methods. For example, restricting the passband window 26 of the received ring signal 22 may allow a sensor 14 with a higher Q to be used, thus providing a longer decay time and higher ring signal 22 amplitude. Restricting the passband window 26 also allows for receiver antennas 24 and filters having a higher Q to be used, thus increasing signal to noise ratio. Further, in systems that utilize a fixed-frequency excitation pulse 20, the sensor's transfer function roll-off dictates that the ring signal 22 may be weaker when the sensor 14 is near the edges of its operational frequency range. Adapting the reader's circuitry to focus on bands near the edges may compensate for this effect.

Once the passband window 26 has been determined, many of the reader's internal components may be tuned to focus only on the range of the passband window 26. For example, the reader's receive antenna 24 may be tuned to the passband window 26 containing the ring signal 22. This may be accomplished by switching reactive components in and out of the antenna circuit, including parts of the antenna 24, or by other methods known in the art.

The wireless system 10 may include an amplifier section. The amplifier section may include filters and amplifiers. The filters and amplifiers may be adaptively tuned to the frequency passband window 26 that contains the ring signal 22. This can be accomplished by switching reactive components in and out of the amplifier and filter circuits, or by other methods known in the art.

The wireless system 10 may include at least one phase lock loop (PLL) to lock onto and help determine the ring frequency. The initial reference frequency for the PLL may be set to approximately the center of the frequency passband window 26. This will reduce the time it takes for the PLL to lock onto the ring signal 22 frequency. For example, the reader 12 processor may calculate or look up the control voltage of the PLL's voltage controlled oscillator (VCO) that corresponds to the center of the passband window 26, as defined by the reader's ambient pressure sensor 25. Other methods and circuits for locking and pre-locking the PLL may be used in conjunction with the systems and methods described herein.

The excitation pulse 20 emitted by the reader 12 may be held at an approximately fixed frequency. The fixed excitation pulse 20 may be adapted to be located near the center of the passband window 26 containing the ring signal 22. As a result, the system may utilize a sensor 14 having a higher Q that may provide a stronger, longer lasting ring signal 22.

The wireless system 10 may utilize a swept frequency excitation pulse 20. The bandwidth of the swept frequency excitation pulse 20 may be limited to the passband window 26 containing the ring signal 22. Limiting the excitation pulse 20 in this manner may reduce the time required to acquire the ring signal 22 and allow more samples to be taken for a given pressure instance.

The parameter measured by the sensor 14 may be static or quasi-static in comparison to the speed of measurement. By way of a non-limiting example, a measured blood pressure waveform may be static or quasi-static in comparison to the speed of measurement. In such circumstances, the reader 12 may take multiple readings of the sensor 14 measurement and average them using a processing algorithm. For example, as the ring signal 22 gets weaker and the signal-to-noise ratio (SNR) decreases, the number of noisy, spurious readings may increase. The reader 12 may be configured to ignore any measurements that lie outside the passband window 26 during the averaging process to remove outlying and inaccurate data.

The reader 12 may sample the incoming ring signal 22 and compare the input data with the passband window 26. Based on the comparison, the input data from the ring signal 22 may be stored or discarded. The reader 12 may also optimize or enhance processing of the signal, for example with FFT methods, by only processing portions of the signal that are within the allowed frequency band based on the filtered passband window 26. Other methods of improving the measurement of the received signal based on narrowing the allowed frequency band to match the ambient measurement may also be utilized.

The examples used herein are directed to an ambient pressure reading to determine a narrowed bandwidth for the absolute reading and adapt the reader 12 circuitry and/or algorithms to that bandwidth. It will be appreciated, however, that this method may be used in any circumstance where two sensor measurements are taken and the result of one measurement can be used to limit the possible outcomes of the other measurement. The sensed parameter is not limited to pressure but may be any parameter. Further, the wireless sensors 14 and ambient sensor do not necessarily have to measure the same quantity or parameter but may instead measure different quantities or parameters.

Although the embodiments of the present invention have been illustrated in the accompanying drawings and described in the foregoing detailed description, it is to be understood that the present invention is not to be limited to just the embodiments disclosed, but that the invention described herein is capable of numerous rearrangements, modifications and substitutions without departing from the scope of the claims hereafter. The claims as follows are intended to include all modifications and alterations insofar as they come within the scope of the claims or the equivalent thereof.

## Claims

1. A wireless sensor reader (12) comprising:
a circuit configured to define a set of all possible values of a wireless sensor's (14) frequency output;
a transmit circuit configured to generate an excitation pulse (20) to cause said wireless sensor (14) to emit a signal (22) having a frequency that is proportionate to at least one sensed parameter;
at least one antenna (24) configured to transmit said excitation pulse (20) and receive said emitted signal (22);
a first circuit configured to generate a count signal; and
a second circuit configured to adjust the frequency of said count signal to match the frequency of said emitted signal (20);
wherein said reader (12) is configured to hold said count signal temporarily constant to ascertain said frequency of said count signal;
wherein said circuit for identifying said set of possible frequency values comprises a second sensor (25) that measures a parameter related to the parameter being measured by said wireless sensor (14); and
wherein said second sensor (25) is an ambient pressure sensor.

2. The wireless sensor reader (12) of claim 1, wherein said second circuit comprises a phase-locked loop circuit capable of being placed in a sample mode to receive said emitted signal (22) and adjust the frequency of said count signal based on the frequency of said emitted signal (22), and further wherein said phase-locked loop circuit is capable of being placed in a hold mode to hold the frequency of said count signal constant for a length of time sufficient to determine the frequency of said count signal.

3. The wireless sensor reader (12) of claim 1, wherein said wireless sensor (14) is a blood pressure sensor.

4. The wireless sensor reader (12) of claim 1, wherein said circuit for identifying a set of possible frequency values of said signal identifies a set of likely frequency values of said signal for an individual reading.

5. The wireless sensor reader (12) of claim 1, wherein said antenna (24) is capable of being tuned to transmit an excitation pulse (20) having a frequency that is selected based on said set of possible frequency values of said signal.

6. The wireless sensor reader (12) of claim 1, wherein said antenna is capable of being tuned to receive frequencies in a passband (26) based on said set of possible frequency values.

7. The wireless sensor reader (12) of claim 1, wherein said reader includes circuitry that comprises filters capable of being tuned to reject frequencies outside of a passband (26) based on said set of possible frequency values.

8. The wireless sensor reader (12) of claim 7, wherein said filters comprise digital filters.

9. The wireless sensor reader (12) of claim 8, wherein said digital filters comprise averaging a set of discrete samples.

10. The wireless sensor reader (12) of claim 7, wherein said reader comprises circuitry for signal processing of discrete samples of said frequency of said emitted signal (20).

11. The wireless sensor reader (12) of claim 1, wherein said first circuit selects said count signal's initial frequency value based on said set of possible frequency values.

12. The wireless sensor reader (12) of claim 1, wherein said circuit for identifying a set of possible frequency values of said signal includes an algorithm.

13. The wireless sensor reader (12) of claim 1, wherein said second circuit optimizes said adjustment of said count signal based on said set of possible frequency values.

14. The wireless sensor reader (12) of claim 1, wherein said reader selects the frequency of said excitation pulse (20) based on said set of possible frequency values.

## Patentansprüche

1. Drahtloser Sensor-Leser (12), umfassend:
eine Schaltung, welche dafür konfiguriert ist, einen Satz aller möglichen Werte eines Frequenzausgangs eines drahtlosen Sensors (14) zu definieren;
eine Sendeschaltung, welche dafür konfiguriert ist, einen Anregungspuls (20) zu erzeugen, um den drahtlosen Sensor (14) zum Ausgeben eines Signals (22) zu veranlassen, welches eine Frequenz hat, die mindestens einem abgetasteten Parameter entspricht;
mindestens eine Antenne (24), welche dafür konfiguriert ist, den Anregungspuls (20) zu senden und das ausgegebene Signal (22) zu empfangen;
eine erste Schaltung, welche dafür konfiguriert ist, ein Zählsignal zu erzeugen; und
eine zweite Schaltung, welche dafür konfiguriert ist, die Frequenz des Zählsignals so einzustellen, dass sie der Frequenz des ausgegebenen Signals (20) entspricht;
wobei der Leser (12) dafür konfiguriert ist, das Zählsignal vorübergehend konstant zu halten, um die Frequenz des Zählsignals zu ermitteln;
wobei die Schaltung zum Definieren des Satzes möglicher Frequenzwerte einen zweiten Sensor (25) umfasst, welcher einen Parameter misst, der dem durch den drahtlosen Sensor (14) gemessenen Parameter zugehörig ist; und
wobei der zweite Sensor (25) ein Umgebungsdrucksensor ist.

2. Drahtloser Sensor-Leser (12) nach Anspruch 1, wobei die zweite Schaltung eine PLL-Schaltung enthält, welche dafür geeignet ist, in einen Abtastmodus versetzt zu werden, um das ausgegebene Signal (22) zu empfangen und die Frequenz des Zählsignals basierend auf der Frequenz des ausgegebenen Signals (22) einzustellen, und wobei ferner die PLL-Schaltung dafür geeignet ist, in einen Haltemodus versetzt zu werden, um die Frequenz des Zählsignals für eine Zeitdauer zu halten, die zum Ermitteln der Frequenz des Zählsignals ausreichend ist.

3. Drahtloser Sensor-Leser (12) nach Anspruch 1, wobei der drahtlose Sensor (14) ein Blutdrucksensor ist.

4. Drahtloser Sensor-Leser (12) nach Anspruch 1, wobei die Schaltung zum Definieren eines Satzes möglicher Frequenzwerte des Signals einen Satz wahrscheinlicher Frequenzwerte des Signals für ein individuelles Auslesen definiert.

5. Drahtloser Sensor-Leser (12) nach Anspruch 1, wobei die Antenne (24) dafür geeignet ist, so abgestimmt zu werden, dass sie einen Anregungspuls (20) sendet, welcher eine Frequenz hat, die basierend auf dem Satz möglicher Frequenzwerte des Signals gewählt ist.

6. Drahtloser Sensor-Leser (12) nach Anspruch 1, wobei die Antenne (24) dafür geeignet ist, so abgestimmt zu werden, dass sie Frequenzen innerhalb eines auf dem Satz möglicher Frequenzwerte basierenden Durchlassbereichs (26) empfängt.

7. Drahtloser Sensor-Leser (12) nach Anspruch 1, wobei der Leser Schalttechnik enthält, welche Filter umfasst, die dafür geeignet sind, so abgestimmt zu werden, dass sie Frequenzen außerhalb eines auf dem Satz möglicher Frequenzwerte basierenden Durchlassbereichs (26) abweisen.

8. Drahtloser Sensor-Leser (12) nach Anspruch 7, wobei die Filter digitale Filter umfassen.

9. Drahtloser Sensor-Leser (12) nach Anspruch 8, wobei die digitalen Filter das Mitteln eines Satzes diskreter Abtastwerte umfassen.

10. Drahtloser Sensor-Leser (12) nach Anspruch 7, wobei der Leser Schalttechnik für die Signalverarbeitung diskreter Abtastwerte der Frequenz des ausgegebenen Signals (20) enthält

11. Drahtloser Sensor-Leser (12) nach Anspruch 1, wobei die erste Schaltung den Ausgangsfrequenzwert des Zählsignals basierend auf dem Satz möglicher Frequenzwerte wählt.

12. Drahtloser Sensor-Leser (12) nach Anspruch 1, wobei die Schaltung zum Definieren eines Satzes möglicher Frequenzwerte des Signals einen Algorithmus enthält.

13. Drahtloser Sensor-Leser (12) nach Anspruch nach Anspruch 1, wobei die zweite Schaltung das Einstellen des Zählsignals basierend auf dem Satz möglicher Frequenzwerte optimiert.

14. Drahtloser Sensor-Leser (12) nach Anspruch 1, wobei der Leser die Frequenz des Anregungspulses (20) basierend auf dem Satz möglicher Frequenzwerte wählt.

## Revendications

1. Lecteur à capteur sans fil (12) comprenant :
un circuit configuré pour définir un ensemble de toutes les valeurs possibles d'une sortie de fréquence du capteur sans fil (14) ;
un circuit de transmission configuré pour générer une impulsion d'excitation (20) afin d'amener ledit capteur sans fil (14) à émettre un signal (22) ayant une fréquence qui est proportionnelle à au moins un paramètre détecté ;
au moins une antenne (24) configurée pour transmettre ladite impulsion d'excitation (20) et recevoir ledit signal émis (22) ;
un premier circuit configuré pour générer un signal de comptage ; et
un second circuit configuré pour ajuster la fréquence dudit signal de comptage afin de correspondre à la fréquence dudit signal émis (20) ;
dans lequel ledit lecteur (12) est configuré pour maintenir le signal de comptage temporairement constant afin de vérifier ladite fréquence dudit signal de comptage ;
dans lequel ledit circuit pour identifier ledit ensemble de valeurs de fréquence possibles comprend un second capteur (25) qui mesure un paramètre lié au paramètre qui est mesuré par ledit capteur sans fil (14) ; et
dans lequel ledit second capteur (25) est un capteur de pression ambiante.

2. Lecteur à capteur sans fil (12) selon la revendication 1, dans lequel ledit second circuit comprend un circuit en boucle à verrouillage de phase capable d'être placé dans un mode d'échantillonnage pour recevoir ledit signal émis (22) et ajuster la fréquence dudit signal de comptage en fonction de la fréquence dudit signal émis (22), et dans lequel ledit circuit en boucle à verrouillage de phase est en outre capable d'être placé dans un mode de maintien pour maintenir la fréquence dudit signal de comptage constante pendant une durée suffisante pour déterminer la fréquence dudit signal de comptage.

3. Lecteur à capteur sans fil (12) selon la revendication 1, dans lequel ledit capteur sans fil (14) est un capteur de pression sanguine.

4. Lecteur à capteur sans fil (12) selon la revendication 1, dans lequel ledit circuit pour identifier un ensemble de valeurs de fréquence possibles dudit signal identifie un ensemble de valeurs de fréquence probables dudit signal pour une lecture individuelle.

5. Lecteur à capteur sans fil (12) selon la revendication 1, dans lequel ladite antenne (24) est capable d'être accordée pour transmettre une impulsion d'excitation (20) ayant une fréquence qui est sélectionnée en fonction dudit ensemble de valeurs de fréquence possibles dudit signal.

6. Lecteur à capteur sans fil (12) selon la revendication 1, dans lequel ladite antenne est capable d'être accordée pour recevoir des fréquences dans une bande passante (26) en fonction dudit ensemble de valeurs de fréquence possibles.

7. Lecteur à capteur sans fil (12) selon la revendication 1, dans lequel ledit lecteur inclut une circuiterie qui comprend des filtres capables d'être accordés pour rejeter des fréquences en dehors d'une bande passante (26) en fonction dudit ensemble de valeurs de fréquence possibles.

8. Lecteur à capteur sans fil (12) selon la revendication 7, dans lequel lesdits filtres comprennent des filtres numériques.

9. Lecteur à capteur sans fil (12) selon la revendication 8, dans lequel lesdits filtres numériques comprennent établir la moyenne d'un ensemble d'échantillons discrets.

10. Lecteur à capteur sans fil (12) selon la revendication 7, dans lequel ledit lecteur comprend une circuiterie pour le traitement de signal d'échantillons discrets de ladite fréquence dudit signal émis (20).

11. Lecteur à capteur sans fil (12) selon la revendication 1, dans lequel ledit premier circuit sélectionne la valeur de fréquence initiale dudit signal de comptage en fonction dudit ensemble de valeurs de fréquence possibles.

12. Lecteur à capteur sans fil (12) selon la revendication 1, dans lequel ledit circuit pour identifier un ensemble de valeurs de fréquence possibles dudit signal inclut un algorithme.

13. Lecteur à capteur sans fil (12) selon la revendication 1, dans lequel ledit second circuit optimise ledit ajustement dudit signal de comptage en fonction dudit ensemble de valeurs de fréquence possibles.

14. Lecteur à capteur sans fil (12) selon la revendication 1, dans lequel ledit lecteur sélectionne la fréquence de ladite impulsion d'excitation (20) en fonction dudit ensemble de valeurs de fréquence possibles.
